# DEMANDE DE BREVET EUROPEEN

(11) **EP 1 103 621 A1**
(43) Date de publication de la demande: **30.05.2001**
(21) Numéro de dépôt: 00403276.9
(22) Date de dépôt: 23.11.2000
(51) Int. Cl.: C12Q 1/14, C12Q 1/34, C12Q 1/42

(54) **Methode d'identification du pneumocoque**

(30) Priorité: 25.11.1999 FR 9914847
(71) Demandeur: Stago International, 92600 Asnières (FR)
(72) Inventeur: Contant, Geneviève, 92400 Courbevoie (FR); Beaupere, Francoise, 75017 Paris (FR)
(74) Mandataire: Warcoin, Jacques

(57) **Abrégé**

L'invention concerne un procédé d'identification rapide de *Streptococcus pneumoniae* dans des échantillons biologiques et le kit correspondant. Ce procédé consiste en un méthode en tube gel, dans laquelle l'échantillon est introduit dans une phase gélifiée, dite de révélation, contenant :
- un substrat S₁ spécifique de la neuraminidase induisant en présence de celle-ci une variation de mesure optique détectable, et,
- un substrat S₂ spécifique de la phosphatase, induisant en présence de celle-ci une variation de mesure optique détectable distincte et indépendante de celle induite par S₁.

## Description

L'invention concerne un procédé d'identification rapide de *Streptococcus pneumoniae* (pneumocoque) dans des échantillons biologiques et le kit correspondant.

Les infections à pneumocoque sont fréquentes et sont responsables d'une morbidité et d'une mortalité élevées. L'incidence annuelle des pneumonies à pneumocoque serait de 132 000 cas avec 12 000 décès en France environ (4).

*Streptococcus pneumoniae* est un germe commensal des voies aériennes supérieures qui peut être responsable de pneumopathies communautaires, d'otites aiguës moyennes, de méningites, de bactériémies et d'endocardites. Il atteint les personnes âgées et les jeunes enfants surtout (8).

Sa virulence est liée à la présence d'une capsule polyosidique qui s'oppose à la phagocytose. Les signes cliniques manquent souvent et exposent à un retard diagnostique et à une antibiothérapie différée.

L'identification du pneumocoque est orientée par ses caractères phénotypiques : aspect des colonies, morphologie cellulaire, sensibilité à l'optochine et lyse par la bile (10,13). La réponse par le test à l'optochine n'est disponible qu'en 48 h et l'interprétation est parfois délicate (5). L'identification est compliquée par l'interférence d'autres espèces commensales (7). De plus, certaines espèces comme *S. mitis* et *S. oralis* possèdent des gènes d'enzymes similaires à ceux de *Streptococcus pneumoniae* (11). La détermination des antigènes capsulaires par le test d'agglutination des particules de latex est rapide et simple mais elle présente des faux négatifs et des faux positifs.

L'identification du pneumocoque peut être confirmée par la détermination du sérotype capsulaire à l'aide d'anticorps ou par hybridation spécifique avec la sonde AccuProbe *Streptococcus pneumoniae ®.* Néanmois, l'émergence de pneumocoques de sensibilité diminuée à la pénicilline (PSDP) et résistants aux macrolides et de souches multirésistantes pose un véritable problème de santé publique (2, 6).

Il existe donc aujourd'hui un besoin d'un test rapide et facilement réalisable pour identifier le pneumocoque et le différencier des autres streptocoques oraux directement à partir de prélèvements.

L'invention propose un procédé permettant de détecter rapidement *Streptococcus pneumoniae :*
- à partir de colonies isolées de streptocoques α hémolytiques sur gélose au sang (en 1 heure ou moins),
- directement à partir des bouillons d'hémoculture positifs à cocci à Gram positif dont l'aspect évoque un streptocoque ou un pneumocoque (en 1 heure ou moins),
- directement à partir des prélèvements broncho pulmonaires et des expectorations (en 2 heures environ),
et de le différencier des autres streptocoques en 2 heures.

Le procédé selon l'invention utilise le système gélifié de détection de microorganismes tel que décrit dans WO96/29427, un tel système étant schématiquement constitué d'un tube comprenant au moins une phase dite de révélation, constituée d'un gel comportant un milieu de culture de microorganismes et un réactif induisant une variation de mesure optique détectable en présence de microorganismes. Un tel système gélifié sera ci-après dénommé "tube gel" ou "tube". Les tubes gel préférentiellement utilisés dans le cadre de l'invention sont des tubes dont le fond a une partie capillaire contenant la phase de révélation. Ceci permet de mieux concentrer localement les bactéries donc de mieux visualiser la variation de mesure optique située à ce niveau.

Le principe du procédé selon l'invention est basé sur un diagnostic visuel différentiel. Il consiste en effet à révéler deux activités enzymatiques différentes, l'une permettant l'identification présomptive du pneumocoque, l'autre sa différenciation d'autres streptocoques oraux.

Pour ce faire, le procédé de l'invention utilise deux révélateurs aux propriétés optiques différentes inclus dans le gel, chaque révélateur étant un substrat spécifique de l'une ou l'autre activité enzymatique.

Les pneumocoques sécrètent différents produits, dont notamment des enzymes, en rapport ou non avec leur virulence. Ainsi, les pneumocoques capsulés et fraîchement isolés des malades atteints de méningites et de pneumonies élaborent six glycosidases dont une neuraminidase (sialidase) sécrétée pendant la phase logarithmique de croissance. Cette enzyme a un rôle dans le pouvoir pathogène et invasif du pneumocoque, en clivant l'acide sialique des glycolipides et glycoprotéines des membranes cellulaires (14).

Cette enzyme n'est cependant pas spécifique du pneumocoque, et peut être également synthétisée par d'autres espèces de streptocoques du groupe viridans (3). Ainsi, la détection de son activité dans un échantillon biologique dans lequel on suspecte la présence de *Streptococcus pneumoniae* permet seulement une identification présomptive, qui doit être confirmée ou infirmée par un test complémentaire.

Le procédé selon l'invention consiste donc à associer la détection d'une activité sialidase à celle d'une seconde activité enzymatique connue pour être présente chez les autres streptocoques oraux (les *Streptococcus viridans)* et absente chez le pneumocoque. La phosphatase est une enzyme détectée dans les galeries d'identification décrites et évaluées dans la littérature, elle répond à de tels critères (15, 16).

La présente invention a donc pour objet une méthode d'identification en tube gel de *Streptococcus pneumoniae* dans un échantillon biologique, dans laquelle l'échantillon est introduit dans une phase gélifiée, dite de révélation, contenant :
- un substrat S₁ spécifique de la neuraminidase induisant en présence de celle-ci une variation de mesure optique détectable, et,
- un substrat S₂ spécifique de la phosphatase induisant en présence de celle-ci une variation de mesure optique détectable distincte et indépendante de celle induite par S1.

L'existence d'une activité neuraminidase, révélée par une variation de mesure optique induite par S1 donne lieu à une identification présomptive de *Streptococcus pneumoniae.* L'existence ou l'absence d'une activité phosphatase associée, révélée par une variation de mesure optique induite par S2, permet de différencier *Streptococcus pneumoniae* d'autres streptocoques oraux susceptibles d'exprimer la neuraminidase.

L'invention a également pour objet un kit pour la mise en oeuvre de la méthode telle que définie ci-dessus.

On entend par :
- "échantillon biologique" : les colonies isolées de streptocoques α hémolytiques sur gélose au sang, les bouillons d'hémoculture positifs à cocci à Gram positif dont l'aspect évoque un streptocoque ou un pneumocoque, les prélèvements broncho-pulmonaires.
- "tube gel" ou "tube" : un tube comprenant au moins une phase dite de révélation, constituée d'un gel comportant un milieu de culture de microorganismes et au moins un réactif induisant une variation de mesure optique détectable en présence de microorganismes. Une description détaillée de ces tubes et de leur protocole d'utilisation est accessible à l'homme du métier notamment dans WO96/29427.
- "substrat induisant une variation de mesure optique détectable" : un produit qui sous l'action d'une enzyme spécifique provoque un changement de couleur du milieu, apprécié soit à l'oeil nu, soit au moyen d'un appareil. Le substrat peut ainsi produire un changement de couleur dans le spectre visible, ou un changement de fluorescence ou de luminescence.
- "variation de mesure optique distincte et indépendante" : une variation de mesure optique produite par l'un des substrats qui se différencie de celle produite par le second, qui n'a pas d'interférence avec celle-ci et se lit indépendamment.

Dans la méthode selon l'invention, on détecte la présence ou l'absence de *Streptococcus pneumoniae* dans l'échantillon en procédant à la lecture de la variation de mesure optique induite par S₁ et/ou S₂ dans la phase de révélation.

Plus précisément, on détecte la présence de *Streptococcus pneumoniae* par l'observation d'une variation de mesure optique induite par S₁ et l'absence de variation de mesure optique induite par S₂.

On utilise préférentiellement un substrat fluorogène (S1) et un substrat chromogène (S2).

Avantageusement, le substrat fluorogène est celui de la neuraminidase et le substrat chromogène celui de la phosphatase. Dans ce cas, le substrat de la neuraminidase est par exemple l'acide 4-Methylumbelliferyl-α-D-N-Acétylneuraminique (MUN) ou l'un de ses sels, dont l'utilisation pour la détection d'une activité neuraminidase a été décrite dans la littérature (17, 18). Sa concentration finale dans la phase gel du tube est avantageusement comprise entre 0,25 et 0,5 mM/ml de gel. Préférentiellement cette concentration est de 0,5 mM/ml de gel soit 13 µM/tube gel.

Le substrat chromogène de la phosphatase est choisi parmi les composés utilisés en routine pour détecter cette activité enzymatique tel que par exemple le paranitrophényl phosphate (PNP ou Sigma 104® phosphatase substrate. Ce substrat est utilisé pour les dosages de phosphatase acide et alcaline par méthode colorimétrique). La concentration finale de ce composé dans la phase gel est avantageusement comprise entre 1,5 et 2,5 mM/ml de gel. Elle est préférentiellement égale à 2 mM/ml gel soit 50 µM/tube gel.

La lecture est réalisée dans la partie capillaire du tube-gel, après ensemencement de l'échantillon (dépôt de l'échantillon dans le tube et concentration des bactéries dont le pneumocoque dans la phase de révélation par centrifugation) et incubation du tube gel à 37° C, au moins pendant 10 minutes et préférentiellement pendant 1 à 2 heures, selon le type d'échantillon analysé (voir ci-après).

Plus précisément, lorsque l'échantillon est constitué par des colonies isolées ou des bouillons d'hémoculture dont l'inoculum est défini, la variation de mesure optique induite par S1 (identification présomptive de *Streptococcus pneumoniae* par la révélation de l'activité neuraminidase) est avantageusement lue après 1 heure d'incubation du tube à 37° C. La variation de mesure optique induite par S₂ (test de différenciation par la révélation de l'activité phosphatase) est préférentiellement lue après 1 heure d'incubation supplémentaire, soit 2 heures d'incubation à 37° C.

Dans ces conditions de durée et de température d'incubation, le seuil de détection des bactéries a été trouvé à 10⁸ bactéries/ml, que ce soit avec S₁ *(Streptococcus pneumoniae)* ou avec S₂ *(Streptococcus viridans).* La réaction est douteuse à 10⁷ bactéries/ml et faussement négative à moins de 10⁷ bactéries/ml.

Ainsi dans le cas des colonies isolées, le nombre de colonies doit être suffisant pour que la concentration de l'inoculum soit supérieure ou égale à 10⁸ bactéries/ml. L'inoculum est ajusté par rapport au standard 0,5 de Mac Farland, équivalent en turbidité à 10⁸ bactéries/ml. Ce standard permet une standardisation de l'inoculum d'un test à un autre.

Dans le cas des bouillons d'hémoculture, la numération des cocci gram positif est de 10⁷ à 10¹⁰ bactéries/ml dans un bouillon décanté (on laisse déposer le sang). Le volume d'échantillon doit être choisi pour obtenir une concentration optimale des bactéries avec une interférence minimale des globules rouges. Le meilleur compromis a été obtenu en utilisant un volume de 0,2 ml de bouillon décanté (soit 2.10⁶ à 2.10⁹ bactéries) auquel on ajoute 1 ml d'eau distillée stérile pour lyser les globules rouges et libérer l'hémoglobine dans le surnageant situé au-dessus du gel du tube. Préférentiellement, la concentration de l'inoculum ensemencé dans le tube est supérieure ou égale à 2.10⁶ bactéries/ml.

Le seuil de détection, trouvé à 10⁶ bactéries/ml en bouillon d'hémoculture est plus faible qu'en système purifié (colonies isolées). On peut l'expliquer par une plus grande vitalité des bactéries dans un prélèvement que dans un système purifié.

Lorsque l'échantillon est constitué par des prélèvements broncho-pulmonaires ou des expectorations, les deux variations de mesure optique sont préférentiellement lues après 2 heures d'incubation du tube à 37° C. Il est en effet préférable pour ce type d'échantillon de réaliser un temps d'incubation plus long avant de procéder à l'identification présomptive de *Streptococcus pneumoniae* car, par rapport aux colonies et bouillons d'hémoculture, les volumes d'échantillon déposés dans les tubes-gel sont généralement plus faibles (50 µl au lieu de 200 µl à 1 ml). Ces plus faibles quantités d'échantillon permettent d'éliminer au niveau de la lecture les interférences liées à la présence d'une éventuelle flore contaminante. Il en résulte toutefois une quantité diminuée de bactéries introduites dans le gel, et donc généralement la nécessité d'un temps d'incubation prolongé pour une lecture correcte des tubes.

La difficulté d'analyse des prélèvements broncho-pulmonaires est liée à leur contamination par la salive. Les crachats sont classés, en fonction de la présence de cellules épithéliales et de leucocytes, pour témoigner de leur acceptabilité pour l'examen bactériologique. On se limite habituellement à l'identification et à l'antibiogramme d'une ou deux espèces prédominantes, lorsqu'elles atteignent ou dépassent 10⁷ bactéries/ml.

Le volume d'échantillon a donc été choisi pour obtenir une concentration optimale de la bactérie prédominante à laquelle on accorde une signification clinique avec une interférence minimale des bactéries de la flore, non pathogènes. Le meilleur compromis a été obtenu avec un inoculum de 50 µl, donc 20 fois inférieur à celui des colonies isolées, soit supérieur ou égal à 5.10⁶ bactéries/ml.

Par contre, il a été nécessaire de prolonger le temps d'incubation de 1 heure à 37° C pour obtenir une réponse similaire à celle du système purifié. Il est possible que les bactéries de la flore associée, les cellules du prélèvement et/ou le mucus des sécrétions retardent la concentration des bactéries dans le gel ayant une signification clinique. Ce retard est visualisé par une zone de déchets présente en surface du gel après centrifugation. Le temps d'incubation nécessaire à une réaction positive est de ce fait un peu plus long.

L'hydrolyse du MUN fait apparaître une fluorescence bleue, qui peut être détectée ou visualisée (mesure non quantitative), par exemple, à l'aide d'une lampe U.V.
Celle du PNP fait apparaître une couleur jaune franche, visible à l'oeil nu.
Les résultats observés s'interprètent de la façon suivante :
- l'absence de fluorescence bleue confirme l'absence de bactéries produisant une neuraminidase, et donc l'absence de *Streptococcus pneumoniae .*
- L'apparition d'une fluorescence bleue témoigne de la présence d'une bactérie produisant une neuraminidase, faisant ainsi suspecter la présence de *Streptococcus pneumoniae.*
- La présence d'une fluorescence associée à l'absence d'une couleur jaune, indiquant l'absence de bactéries produisant de la phosphatase, permet de confirmer la présence de *Streptococcus pneumoniae.*
- Inversement, l'apparition d'une couleur jaune associée à la fluorescence, témoigne de la présence d'une bactérie produisant également une phosphatase et écarte donc un diagnostic de *Streptococcus pneumoniae.*

La présente invention a également pour objet un kit pour la mise en oeuvre de la méthode telle que décrite ci-dessus, comprenant un tube gel dont le fond a une partie capillaire dans laquelle est logée la phase de révélation, caractérisé en ce que ladite phase de révélation contient :
- un substrat S₁ spécifique de la neuraminidase induisant en présence de celle-ci une variation de mesure optique détectable, et,
- un substrat S₂ spécifique de la phosphatase, induisant en présence de celle-ci une variation de mesure optique détectable distincte et indépendante de celle induite par S₁.

Préférentiellement, S₁ est un substrat fluorogène et S2 un substrat chromogène, et plus préférentiellement S₁ est l'acide 4-Methylumbelliferyl-α-D-N-Acétylneuraminique (MUN) ou l'un de ses sels, et S₂ est le paranitrophényl phosphate (PNP).

La présente invention a enfin pour objet l'utilisation de l'acide 4-Methylumbelliferyl-α-D-N-Acetylneuraminique (MUN) ou l'un de ses sels dans une méthode d'identification présomptive de *Streptococcus pneumoniae* dans un échantillon biologique, ledit échantillon biologique étant choisi au sein du groupe constitué par les colonies isolées de streptocoques α hémolytiques sur gélose au sang, les bouillons d'hémoculture positifs à cocci à Gram positif dont l'aspect évoque un streptocoque ou un pneumocoque, les prélèvements broncho-pulmonaires ou les expectorations.

Plus particulièrement, la méthode est réalisée en système tube gel tel que défini précédemment.

Le principe général du mode opératoire de la méthode de dépistage d'un microorganisme dans le système tube-gel est décrit dans WO96/29427, ainsi que dans les notices des kits tels que :
- SCREEN GEL ® Uri: détection des bactéries urinaires,
- SCREEN GEL ® Coli : identification *d'Escherichia coli* en urines et bouillons d'hémoculture,
- HEMOFAST ® MRSA : identification de *staphylococcus aureus* en bouillons d'hémoculture et détection de la sensibilité à la méticilline.

Dans le contexte de la présente invention, on préconise pour l'utilisation de la méthode tube-gel, de traiter les échantillons comme indiqué ci-après :

### • Colonies isolées :

Colonies de cocci à Gram positif dont l'aspect évoque un streptocoque, d'aspect morphologique identique et fraîchement isolées (18-24 h à 37° C) sur gélose Columbia ou Trypticase-soja additionnée de 5 % de sang de mouton ou de sang de cheval, sous CO₂ ou en anaérobiose.

### Préparation de l'inoculum :

- Prélever une dizaine de colonies isolées, d'aspect morphologique identique, à l'aide d'une pipette Pasteur stérile, et les suspendre dans le flacon de milieu de dilution.
- Ajuster cette suspension à 0,5 Mac Farland sur densitomètre ou par rapport à un contrôle de turbidité pour obtenir au moins 10⁸ bactéries/ml.

### Ensemencement du tube :

- Déposer doucement 1 ml de cette suspension dans un tube gel.
- Centrifuger 10 minutes à 3000 g (≃ 4500 TPM) à température ambiante le tube fermé.

### Incubation - lecture :

- Incuber à 37° C le tube fermé pendant 1 heure et lire dans la partie capillaire du tube l'apparition d'une fluorescence bleue franche à l'aide d'une lampe U.V. faisant suspecter la présence de *Streptococcus pneumoniae.* Prolonger l'incubation à 37° C jusqu'à 2 heures et lire l'absence d'une couleur jaune franche visible à l'oeil nu, confirmant la présence de *Streptococcus pneumoniae.*

### • Bouillons d'hémoculture :

Prélever stérilement 0,2 ml de bouillon d'hémoculture contenant des cocci à Gram positif dont l'aspect évoque un streptocoque pour obtenir au moins 2.10⁶ bactéries/ml.
- Déposer doucement 1 ml de milieu de dilution dans un tube gel. Vérifier que le gel n'a pas été perturbé par l'introduction du milieu.
- Ajouter stérilement 0,2 ml de bouillon d'hémoculture à l'aide d'une pipette et 1 ml d'eau distillée stérile.
- Mélanger doucement par retournement.
- Centrifuger le tube 10 minutes à 3000 g(~ 4500 TPM) à température ambiante le tube fermé.
- Incuber le tube fermé à 37° C pendant 1 heure et lire dans la partie capillaire à l'aide d'une lampe à U.V., l'apparition d'une fluorescence bleue en haut de GEL, faisant suspecter la présence de *Streptococcus pneumoniae.* Prolonger l'incubation à 37° C jusqu'à 2 heures et lire à l'oeil nu, l'absence de couleur jaune franche confirmant la présence de *Streptococcus pneumoniae.*

### • Prélèvements broncho-pulmonaires et expectorations :

### Prélèvement :

Expectorations : le recueil doit se faire le matin, au réveil, après rinçage bucco-dentaire à l'eau stérile et lors d'un effort de toux.

Aspirations bronchiques : les techniques sont : le brossage bronchique protégé (BBP), le lavage brochoalvéolaire (LBA) ou les aspirations non protégées.

Aspirations endotrachéales (AET) : l'aspiration des sécrétions par la sonde d'intubation est pratiquée si les deux méthodes invasives ne sont pas réalisées, ainsi que chez le nourrisson.

### Traitement :

Les expectorations doivent être diluées au 1:2 en N-acétylcystéine à 2 % (ou en solution fluidifiante comme Digest ® EUR) et fluidifiés en laissant agir pendant 10 minutes à température ambiante, sous agitation.
- Introduire stérilement 50 µl d'échantillon d'expectoration ou 500 µl de LBA préalablement traité dans le flacon de milieu de dilution. Homogénéiser.

Dans le cas d'un liquide de lavage broncho-alvéolaire (LBA), le dénombrement de germes banals supérieur à 10⁴ bactéries/ml est généralement considéré comme significatif d'une pneumonie. Le volume d'échantillon de 50 µl permet d'obtenir un seuil de détection supérieur à 10⁶ bactéries/ml. Ce volume a donc été augmenté (500 µl au de 50 µl) de façon à permettre d'obtenir un seuil de détection convenable (environ 5.10⁵ bactéries/ml).
- Déposer doucement 1 ml de milieu homogénéisé dans un tube gel.
- Centrifuger 10 minutes à 3000 g (≃ 4500 TMP) à température ambiante le tube fermé.

- Incuber le tube fermé à 37° C.
- Après 2 heures d'incubation, procéder à la lecture du tube, comme indiqué précédemment.

Les exemples ci-après illustrent la présente invention :

### EXEMPLES:

### Exemple n° 1 : Détermination de la spécificité de la méthode.

Le test est réalisé à partir de colonies isolées de cocci Gram positif.

### Matériels et Méthodes :

### 1. Souches :

Les souches sont :
- soit des souches de collection : de l'Institut Pasteur (CIP) ou de la collection américaine (ATCC),
- soit des isolats cliniques provenant de patients de divers hôpitaux français.

Ces souches ont été congelées à - 80° C, en milieu glycérolé après un repiquage sur gélose au sang pour vérifier leur pureté. Elles ont été isolées, 24 heures avant la réalisation du test, sur gélose trypticase-soja, additionnée de 5 % de sang de mouton et incubées à 37° C en présence ou non de CO₂.

| **Souche n°** | **Nom** | **Référence** | **Milieu d'isolement** |
|---|---|---|---|
| 1 | *Streptococcus pneumoniae* | CIP 103566 | GS + CO₂ |
| 2 | *Streptococcus oralis* | CIP 102922 | GS + CO₂ |
| 3 | *Streptococcus sanguis* | CIP 1032231 | GS + CO₂ |
| 4 | *Streptococcus mitis* | CIP 103335 | GS + CO₂ |
| 5 | *Streptococcus pyogenes (A)* | LUQ | GS + CO₂ |
| 6 | *Streptococcus agalactiae (B)* | 53 | GS + CO₂ |
| 7 | *Streptococcus faecalis (D)* | 163 | GS ± CO₂ |
| 8 | *Staphylococcus aureus* | ATCC 25923 | GS |

### 2. Réactifs :

### • Tube gel d'identification du pneumocoque contenant dans sa partie capillaire :

- 25 µl d'une phase gélifiée constituée d'un gel de type Séphadex à une concentration de 50 g/l, gonflé dans un milieu de culture comprenant le substrat MUN à une concentration de 0.5 mM/ml de gel et le substrat PNP à une concentration de 2 mM/ml de gel dans un tampon additionné de conservateurs,
- 20 µl d'une couche protectrice du gel, constituée d'huile de parafine et servant à amortir les chocs lors de l'ensemencement du tube et à éviter le déssèchement du gel.

### • Milieu de dilution :

Il s'agit d'un milieu de culture composé de peptones, d'un sucre en milieu tamponné et d'un agent de lyse des globules rouges. Ce milieu sert à diluer le prélèvement.

### 3. Protocole :

- Réalisation de l'inoculum à 10⁸ bactéries/ml par rapport au standard Mac Farland et dilutions de raison 10 pour avoir : 10⁷ et 10⁶ bactéries/ml (voir protocole indiqué ci-dessus dans la description).
- Ensemencement et lecture des tubes (voir protocole indiqué ci-dessus dans la description).

### Résultats :

| | MUN (Fluorescence bleue) | PNP (couleur jaune) |
|---|---|---|
| *Streptococcus pneumoniae* | *+* | - |
| *Streptococcus oralis* | *+* | *+* |
| *Streptococcus sanguis* | *+* | *+* |
| *Streptococcus mitis* | - | - |
| *Streptococcus pyogenes (A)* | *-* | *+* |
| *Streptococcus agalactiae (B)* | *-* | *+* |
| *Streptococcus faecalis (D)* | - | - |
| *Staphylococcus aureus* | *-* | *+* |

### Conclusion :

Seul *Streptococcus pneumoniae* possède la neuraminidase en l'absence de phosphatase.

### Exemple n° 2 : Détermination de la concentration du substrat MUN à utiliser dans la phase gel.

Le test est réalisé à partir de colonies isolées de cocci Gram positif catalase négative (Streptocoques).

### Matériels et Méthodes :

### 1. Souches :

Elles sont identiques à celles utilisées dans l'exemple n° 1, excepté la souche de *Staphylococcus aureus* (ATCC 25923) qui n'a pas été utilisée dans cet exemple. Les souches sont isolées sur gélose Trypticase-soja, additionnée de 5 % de sang de mouton et incubées à 37° C sous CO₂.

### 2. Réactifs :

Tubes gel d'identification du pneumocoque comprenant différentes concentrations de MUN : 1 mM/ml de gel, 0,5 mM/ml de gel et 0,25 mM/ml de gel. La composition des autres constituants est identique à celle indiquée dans l'exemple n° 1.

### 3. Protocole :

- Réalisation de l'inoculum à 10⁸ bactéries/ml par rapport au standard 0,5 de Mac Farland.
- Ensemencement et lecture des tubes (fluorescence) après 1 heure d'incubation à 37° C.

### Résultats :

| | Concentration de MUN (mM/ml de gel) | | |
|---|---|---|---|
| | 0,25 | 0,5 | 1 |
| *Streptococcus pneumoniae* | *+* (faible) | + | + |
| *Streptococcus mitis* | - | - | - |
| *Streptococcus oralis* | *+* (faible) | + | + |
| *Streptococcus agalactiae* | *-* | - | - |
| *Streptococcus faecalis* | - | - | - |
| *Streptococcus pyogenes* | *-* | - | - |
| *Streptococcus sanguis* | + (faible) | + | + |

### Conclusion :

Pour chaque souche possédant une neuraminidase, la concentration de MUN permettant une détection franche (fluorescence facilement détectable) après 1 h d'incubation à 37° C est de 0,5 mM/ml de gel.

### Exemple n° 3 : Détermination de la concentration de PNP à utiliser dans la phase gel.

Le test est réalisé à partir de colonies isolées de streptocoques.

### Matériels et Méthodes :

### 1. Souches :

Souches de collection.
*Streptococcus pneumoniae* (CM103566)
*Streptococcus oralis (CIP 102922)*

Elles sont isolées sur gélose trypticase déjà additionnée de 5 % de sang de mouton et incubées à 37° C sous CO₂.

### 2. Réactif :

Tubes gel d'identification du pneumocoque comprenant différentes concentrations de PNP : 1,5 mM/ml de gel, 2 mM/ml de gel, 2,5 mM/ml de gel. La composition des autres constituants est identique à celle indiquée dans l'exemple n° 1.

### 3. Protocole :

- Réalisation de l'inoculum à 10⁸ bactéries/ml par rapport au standard 0,5 de Mac Farland et dilutions de raison 10 pour avoir 10⁷ et 10⁶ bactéries/ml.
- Ensemencement et lecture des tubes (couleur jaune visible à l'oeil nu) après 1 h ou 2 h d'incubation à 37° C.

### Résultats :

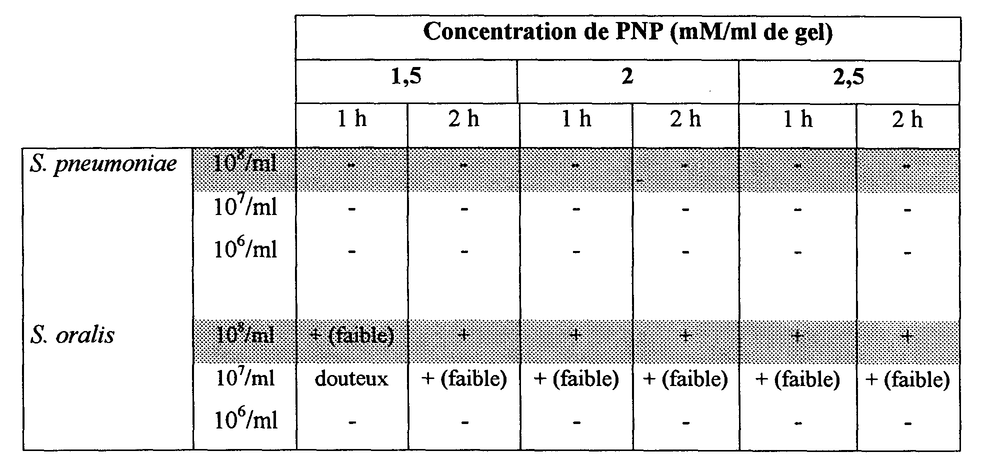

### Conclusion :

- La souche de *Streptococcus pneumoniae* ne possède pas de phosphatase. Elle n'hydrolyse pas le p-nitrophényl-phosphate, quelle que soit la concentration de substrat et le temps d'incubation.
- La souche de *Streptococcus oralis* possède une phosphatase. Sa détection n'est possible que lorsque l'inoculum est supérieur ou égal à 10⁷ bactéries/ml de préférence 10⁸ bactéries/ml. Elle est d'autant plus franche que la concentration en substrat est élevée (> 1,5 mM/ml de gel) et que le temps d'incubation est prolongé (2 heures préférentiellement à 1 heure).

### Exemple n° 4 : Détermination du temps d'incubation nécessaire avant la lecture des tubes gel.

Le test est réalisé à partir de colonies isolées et de bouillons d'hémoculture.

### Matériels et Méthodes :

### 1. Souches :

Les souches de streptocoques (tableau A) sont des isolats cliniques provenant de patients hospitalisés au CHG de Versailles (Le Chesnay). Après identification par la méthode du laboratoire, elles ont été isolées sur gélose trypticase-soja additionnée de 5 % de sang de mouton et incubées à 37° C sous CO₂ pendant 24 h et incubées dans tubes gel pendant des temps variant de 10 minutes à 2 heures avant lecture de la fluorescence (F) du substrat MUN et de la couleur jaune (J) du substrat PAL.

### 2. Bouillons :

Les bouillons d'hémoculture sont des bouillons VITAL® (Biomérieux) trouvés positifs sur l'automate. Ces bouillons contiennent des cocci Gram positif faisant suspecter un streptocoque, à la coloration de Gram. Après décantation, 0,2 ml de bouillon ont été prélevés et ensemencés en tube gel d'identification du pneumocoque.

### 3. Réactifs :

Identiques à ceux indiqués dans l'exemple n° 1.

### 4. Protocole :

- Réalisation de l'inoculum à 10⁸ bactéries/ml par rapport au standard 0,5 de Mac Farland à partir des colonies isolées de streptocoques.
- Prélèvement de 0,2 ml de bouillon décanté auquel on ajoute 1 ml d'eau distillée stérile.
- Ensemencement et lecture des tubes après des temps d'incubation variant de 10 minutes à 2 heures.

### Résultats :

Les temps d'apparition d'une fluorescence (F) avec le substrat MUN et/ou d'une couleur jaune (J) avec le substrat PAL, détectables sans ambiguïté sont indiqués dans les tableaux ci-dessous. Pour les colonies isolées de streptocoques (tableau A), les résultats sont donnés en 2 heures lorsque le test est négatif. Le délai d'apparition de la positivité est indiqué lorsque le test est positif. Pour certains bouillons d'hémoculture (tableau B) deux volumes différents d'échantillon, décanté ou non, ont été testés comparativement. Les résultats sont donnés en 2 heures lorsque le test est négatif. Le délai d'apparition de la positivité est indiqué lorsque le test est positif.

### A/ Colonies isolées de Streptocoques (n = 65) dont Streptococcus pneumoniae (n = 50).

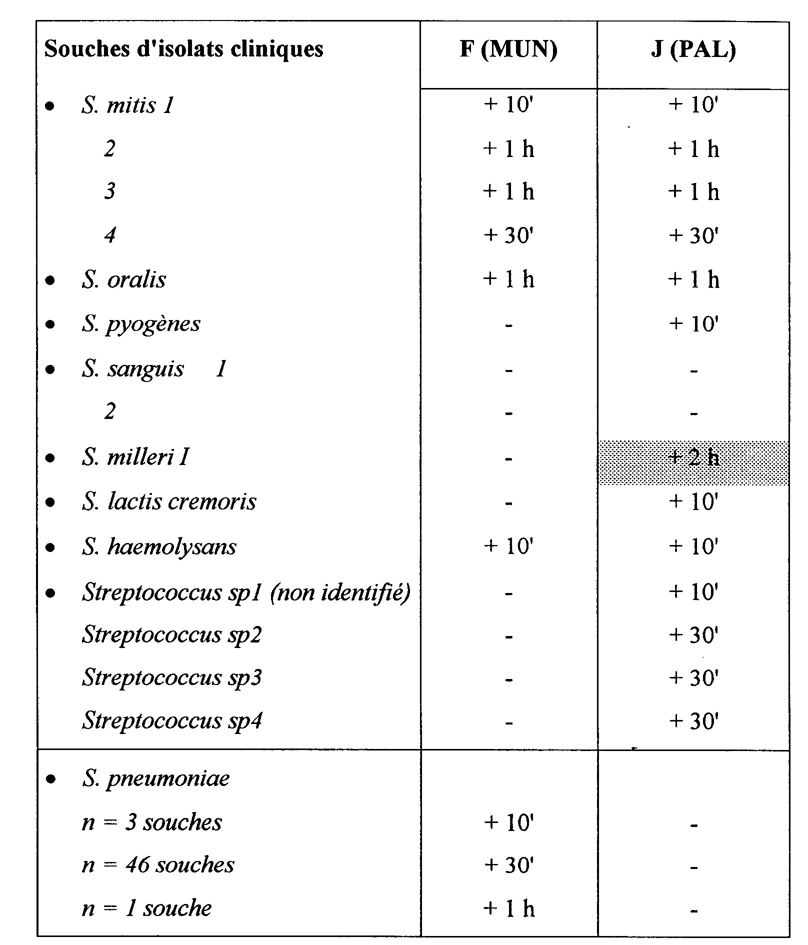

### B/ Bouillons d'hémoculture (n = 9).

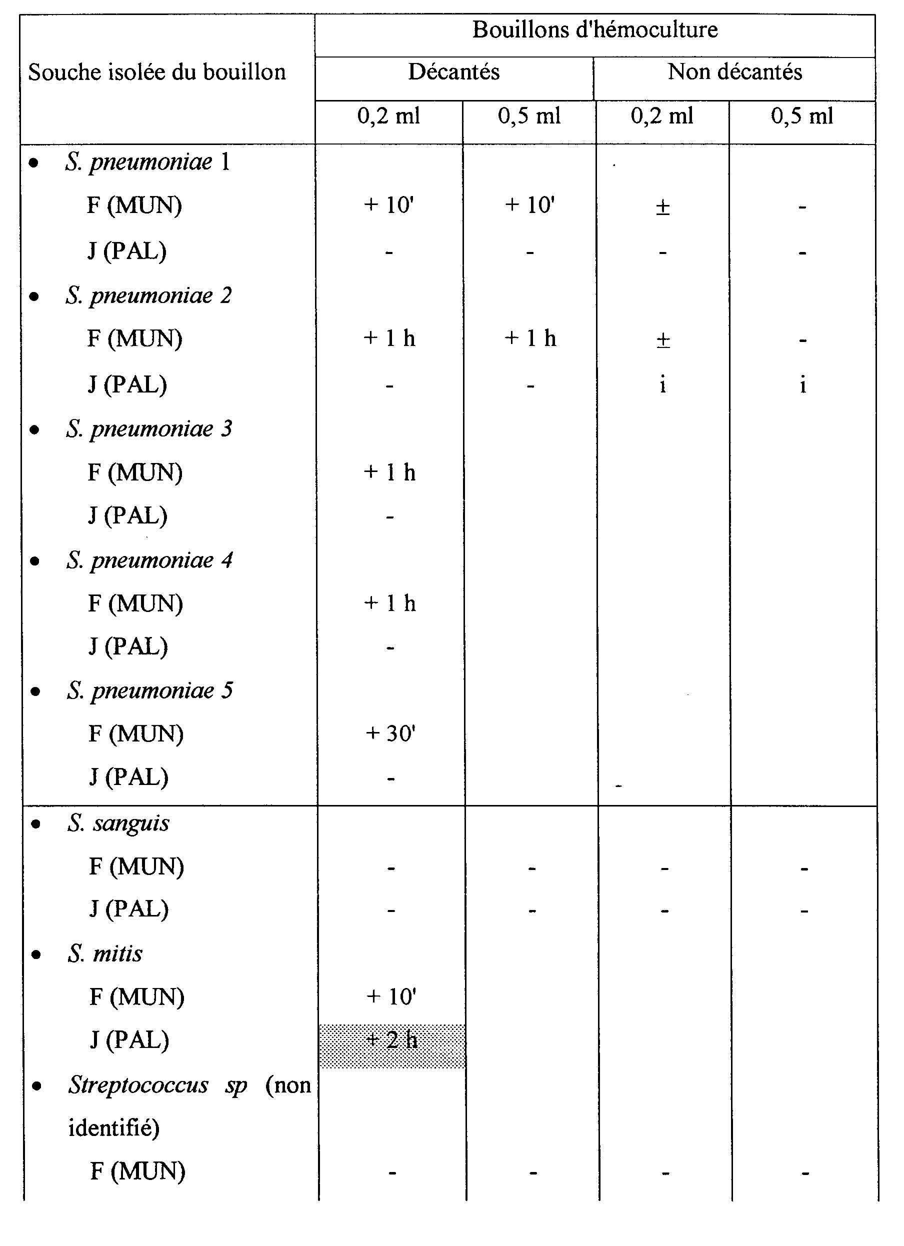

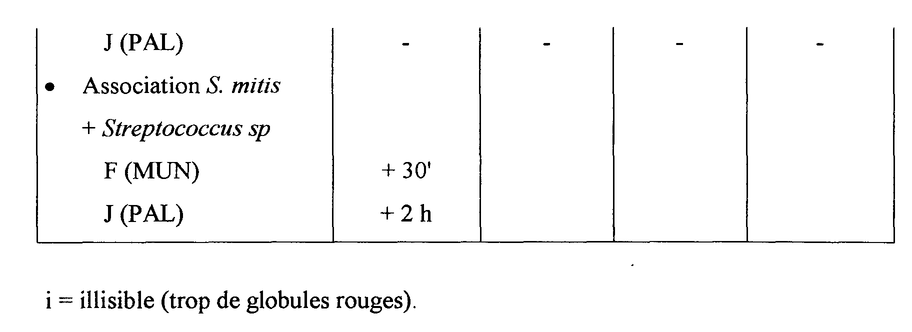

### Conclusion :

Pour s'assurer une bonne sensibilité de détection de l'activité neuraminidase (substrat MUN) ou phosphatase (substrat PAL) : pas de risque de faux négatifs aux concentrations habituellement rencontrées en bactéries, il convient de réaliser la lecture de la fluorescence (F) après 1 h d'incubation et celle de la couleur jaune (J) après 2 h d'incubation.

En effet, une souche de *S. milleri* en système purifié et une souche de *S. mitis* en bouillon d'hémoculture n'ont été positives qu'en 2 h par le test PAL.

D'autre part, il est nécessaire de décanter les bouillons pour éliminer un maximum de globules rouges qui gènent la concentration bactérienne et retardent la réaction. Un volume de bouillon de 0,2 ml est suffisant pour obtenir une réaction positive.

### Exemple n° 5 : Détermination du volume d'aspirations bronchiques à déposer dans le tube-gel pour une bonne détection.

### • Aspiration bronchiques et expectorations :

Le test est réalisé à partir d'aspirations bronchiques prélevées de patients hospitalisés au CHG de Montbéliard. Ces aspirations ont été fluidifiées par le N-acétylcystéine dès réception du prélèvement et congelées à - 20° C après dilution au 1:2 en milieu glycérolé. Il est aussi réalisé à partir d'expectorations prélevées de patients hospitalisés au CHG de Versailles et fluidifiées avant le test.

### • Réactifs :

Identiques à ceux utilisés dans les exemples précédents.

### • Protocole :

Utilisation de volumes variables de prélèvement traité et décongelé à 37° C : 50 à 200 µl ( soit 25 à 100 µl de prélèvement traité) pour ensemencer les tubes. Lecture de la fluorescence et de la couleur jaune après 1 h et 2 h d'incubation à 37° C.

### • Résultats :

Lorsqu'on utilise 200 µl de prélèvement décongelé (soit 100 µl de prélèvement traité), une zone de déchets importante recouvre le gel et gène la concentration des bactéries. De plus, l'hémoglobine libérée par lyse des globules rouges gène la lecture. Les résultats obtenus avec 25 µl et 50 µl sont reportés dans le tableau A ci-dessous, ceux obtenus avec 50 µl sur 50 prélèvements sont reportés dans le tableau B.

### A/ Comparaison des résultats obtenus avec 25 µl et 50 µl de prélèvement (n = 18).

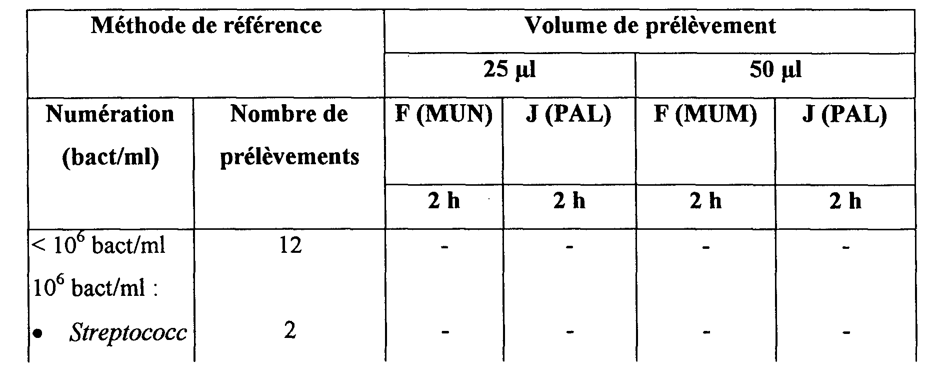

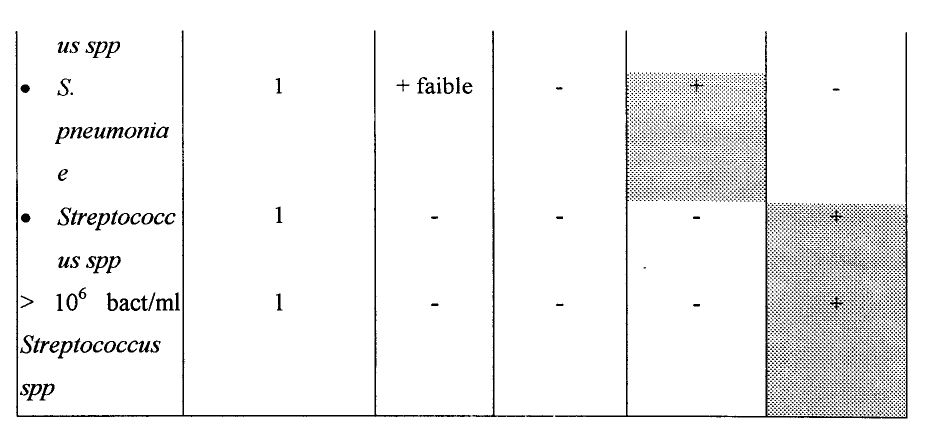

### B/ Résultats obtenus sur 50 prélèvements d'expectorations avec 50 µl d'échantillon :

| **Méthode de référence** | | **Réactions** | |
|---|---|---|---|
| **Numération (bact/ml)** | **Nombre de prélèvements** | **F (MUN)** | **J (PAL)** |
| | | **2h** | **2h** |
| < 10⁶ bact/ml | 33 | - | - |
| | 2* | + | - |
| 10⁶ bact/ml | | | |
| *S. pneumoniae* | *1* | *+* | - |
| ≥ 10⁶ bact/ml | | | |
| • *Staphylococcus sp* | 8 | - | + si ≥ 10⁷/ml |
| • *Streptococcus sp* | 4 | - | *+* |
| • *Staphylococcus sp +* autres | 2 | - | + |
| bactéries | | | |

| | | | |
|---|---|---|---|
| * • *Streptococcus sp* (10⁵/ml) + Staphylococcus sp (10³/ml) • *Streptococcus sp* (10⁵/ml). | | | |

### Conclusion :

- Les réactions de fluorescence (MUN) et de coloration jaune (PAL) sont plus sensibles lorsque la quantité de prélèvement est de 50 µl après 2 h d'incubation à 37° C (tableau A).
- La présence d'une couleur jaune témoigne :
   - de l'association de *Streptococcus pneumoniae* ≥ 10⁶/ml (MUN +) avec une bactérie ayant une phosphatase à concentration ≥ 10⁶ bact/ml (Streptococcus ou Staphylococcus),
   - ou de l'absence de *Streptococcus pneumoniae* ≥ 10⁶/ml (MUN -) avec présence de streptocoques ou de staphylocoques de la flore ≥ 10⁶/ml.
- L'absence de couleur jaune confirme la présence de *Streptococcus pneumoniae >* 10⁶/ml. Deux faux positifs sont apparus avec une faible quantité de streptocoques ayant une neuraminidase (10⁵/ml) mais n'exprimant pas la phosphatase à cette concentration.

Le test effectué sur ce type de prélèvements permet d'éliminer les prélèvements ne contenant pas de pneumocoques ayant une signification clinique. Par contre, il est nécessaire de confirmer la présence d'un pneumocoque lorsque le test de fluorescence est positif.

### REFERENCES

1. ANDRON P., BOUSSOUGANT Y. 1994. *Streptococcus pneumoniae :* données actuelles. Feuillets de biologie, **198**:15-24.
2. BEDOS J.-P., VALLEE E., MOINE P., GESLIN P. et CHASTANG Cl. 1995. Pneumonies à *S. pneumoniae* de sensibilité diminuée à la pénicilline : données épidémiologiques, facteurs de risque et impact thérapeutique. Méd. Mal. Infect. **25**:740-747.
3. BEIGHTON D., HARDIE J.M. and WHILEY R.A. 1991. A scheme for the identification of viridans streptococci. J. Med. Microbiol. **35**:367-372.
4. Communiqué. 1999. Infections à pneumocoque : un pas décisif dans la prévention. La lettre de l'Infectiologue. **1**:41-42.
5. GARDAM M.A., MILLER M.A. 1998. Optochin revisited : defining the optimal type of blood agar for presumptive identification of *Streptococcus pneumoniae.* Jal of Clin. Microbiol. **36**:833-834.
6. GESLIN P., FREMAUX A., SISSIA G. 1993. Situation actuelle de la résistance des pneumocoques aux antibiotiques en France : bilan du Centre National de Référence. La Lettre de l'Infectiologue. **15**:477-486.
7. Groupe Rémic de la Société Française de Microbiologie. 1998. LE REMIC. 1^{ère} édition.. p 43-48.
8. HORAUD T., DAVID F. 1997. Actualités sur les streptocoques oraux : clinique et sensibilité aux antibiotiques. La Lettre de l'Infectiologue. **5**:205-211.
9. LANGLET S., BEAUPERE F., CONTANT G., SCHEFTEL J.-M. 1999. A new gel tube method for the direct detection, identification and susceptibility testing of bacteria in clinical samples. FEMS Microb. Letters. **170**:229-235.
10. LUND E. 1959. Diagnosis of pneumococci by the optochin and bile tests. Acta Path. Microbiol. Scand. **47**:308-315.
11. SCHLEGEL L., BOUVET A. 1998. Streptocoques et genres apparentés abiotrophes et entérocoques. Bull. Soc. Fr. Microbiol. **13**:7-17.
12. THOINET S., BOUCAUD-MAITRE Y. 1993. Analyse des prélèvements en bactériologie médicale. 4^{ème} partie : secrétions bronchopulmonaires. Feuillets de Biologie, Vol XXXIV, **193**:15-18.
13. WASILAUSKAS B.L., HAMPTON K.D. 1984. An analysis of *Streptococcus pneumoniae* identification using biochemical and serological procedures. Diagn. Microbiol. Infect. Dis. **2**:301-307.
14. HORAUD T. et LE BOUGUÉNEC C. 1989. Bactériologie Médicale - Léon Le Minor et Michel Véron, 2^{ème} Ed. Médecine-Sciences Flammarion - Chapître 39 : Streptococcaceae, pp 817-820 : Streptococcus pneumoniae.
15. MOATTI N. et MOUNE A. 1988. "Techniques actuelles d'identification rapide des bactéries isolées en culture (automates exclus). Feuillets de Biologie, Vol. XXIX, **161**:17-25.
16. D'AMATO R. F., McLAUGHLIN J.-C. and FERRARO M.J. 1985. Manual of Clinical Microbiology, Fourth Edition, E.H. LENETTE, ASM Washington D.C., Chapter 6 : "Rapid Manual and Mechanized/Automated Methods for the detection and identification of bacteria and yeasts", pp 52-61.
17. POTIER M., MAMELI L., BELISLE M., DALLAIRE L., and MELANCON S.B. 1979. Fluorometric Assay of Neuraminidase with a Sodium (4-Methylumbelliferyl-α-D-N-Acetylneuraminate) Substrate. Analytical Biochemistry **94**:287-296.
18. BEIGHTON D. and WHILEY R. A. June 1990. Sialidase Activity of the *"Streptococcus milleri* Group" and Other Viridans Group Streptococci. Journal of Clinical Microbiology, Vol 28, **6**:1431-1433.

## Revendications

1. Méthode en tube gel pour la détection de *Streptococcus pneumoniae* dans un échantillon biologique, dans laquelle l'échantillon est introduit dans une phase gélifiée, dite de révélation, contenant :
- un substrat S₁ spécifique de la neuraminidase induisant en présence de celle-ci une variation de mesure optique détectable, et,
- un substrat S₂ spécifique de la phosphatase, induisant en présence de celle-ci une variation de mesure optique détectable distincte et indépendante de celle induite par S₁.

2. Méthode selon la revendication 1, caractérisée en ce que l'on détecte la présence ou l'absence de *Streptococcus pneumoniae* dans l'échantillon en procédant à la lecture de la variation de mesure optique induite par S₁ et/ou S₂ dans la phase de révélation.

3. Méthode selon la revendication 1, caractérisée en ce que la lecture d'une variation de mesure optique induite par S₁ permet une identification présomptive de *Streptococcus pneumoniae.*

4. Méthode selon la revendication 1, caractérisée en ce que la présence de *Streptococcus pneumoniae* est confirmée par la lecture d'une variation de mesure optique induite par S₁ et l'absence de variation de mesure optique induite par S₂.

5. Méthode selon la revendication 1, caractérisée en ce que S₁ est un substrat fluorogène et S₂ un substrat chromogène.

6. Méthode selon la revendication 1, caractérisée en ce que S₁ est l'acide 4-Methylumbelliferyl-α-D-N-Acétylneuraminique (MUN) ou l'un de ses sels, et S₂ est le paranitrophényl phosphate (PNP).

7. Méthode selon la revendication 6, caractérisée en ce que la concentration finale dans le gel de MUN est comprise entre 0,25 et 0,5 mM/ml de gel.

8. Méthode selon la revendication 6, caractérisée en ce que la concentration finale dans le gel de MUN est de 0,5 mM/ml de gel.

9. Méthode selon la revendication 6, caractérisée en ce que la concentration finale dans le gel de PNP est comprise entre 1,5 et 2,5 mM/ml de gel.

10. Méthode selon la revendication 6, caractérisée en ce que la concentration finale dans le gel de PNP est de 2 mM/ml de gel.

11. Méthode selon la revendication 1, caractérisée en ce que l'échantillon est choisi au sein du groupe constitué par les colonies isolées de streptocoques a hémolytiques sur gélose au sang, les bouillons d'hémoculture positifs à cocci à Gram positif dont l'aspect évoque un streptocoque ou un pneumocoque, les prélèvements broncho-pulmonaires.

12. Méthode selon la revendication 11, caractérisée en ce que la concentration de l'inoculum est supérieure ou égale à 10⁸ bactéries/ml lorsque l'échantillon est constitué par des colonies isolées.

13. Méthode selon la revendication 11, caractérisée en ce que la concentration de l'inoculum est supérieure ou égale à 2.10⁶ bactéries/ml lorsque l'échantillon est constitué par un bouillon d'hémoculture décanté.

14. Méthode selon la revendication 11, caractérisée en ce que la concentration de l'inoculum est supérieure ou égale à 5.10⁶ bactéries/ml lorsque l'échantillon est constitué par des prélèvements broncho-pulmonaires.

15. Méthode selon la revendication 6, caractérisée en ce que l'on réalise avant la lecture du tube, une étape d'incubation d'1 heure à 2 heures à 37° C.

16. Méthode selon la revendication 15, caractérisée en ce que la variation de mesure optique induite par S₁ est lue après 1 heure d'incubation du tube à 37° C et celle induite par S₂ après 2 heures d'incubation, lorsque l'échantillon est constitué par des colonies isolées ou des bouillons d'hémoculture.

17. Méthode selon la revendication 15, caractérisée en ce que la variation de mesure optique induite par S₁ et celle induite par S₂ sont lues après 2 heures d'incubation à 37° C, lorsque l'échantillon est constitué par des prélèvements broncho-pulmonaires.

18. Kit pour la mise en oeuvre de la méthode selon l'une quelconque des revendications 1 à 17, comprenant un tube gel dont le fond a une partie capillaire dans laquelle est logée la phase de révélation, caractérisé en ce que ladite phase de révélation contient :
- un substrat S₁ spécifique de la neuraminidase induisant en présence de celle-ci une variation de mesure optique détectable et,
- un substrat S₂ spécifique de la phosphatase, induisant en présence de celle-ci une variation de mesure optique détectable distincte et indépendante de celle induite par S₁.

19. Kit selon la revendication 18, caractérisé en ce que S₁ est un substrat fluorogène et S₂ un substrat chromogène.

20. Kit selon la revendication 18, caractérisé en ce que S₁ est l'acide 4-Methylumbelliferyl-α-D-N-Acétylneuraminique (MUN) ou l'un de ses sels, et S₂ est le paranitrophényl phosphate (PNP).

21. Utilisation de l'acide 4-Methylumbelliferyl-α-D-N-Acetylneuraminique (MUN) ou l'un de ses sels dans une méthode d'identification présomptive de *Streptococcus pneumoniae* dans un échantillon biologique.

22. Utilisation selon la revendication 21, caractérisée en ce que l'échantillon est choisi au sein du groupe constitué par les colonies isolées de streptocoques a hémolytiques sur gélose au sang, les bouillons d'hémoculture positifs à cocci à Gram positif dont l'aspect évoque un streptocoque ou un pneumocoque, les prélèvements broncho-pulmonaires et les expectorations.

23. Utilisation selon la revendication 21, caractérisée en ce que la méthode est réalisée dans un système tube-gel.
